# EUROPEAN PATENT APPLICATION

(11) **EP 4 729 501 A1**
(43) Date of publication of application: **22.04.2026**
(21) Application number: 25306691.4
(22) Date of filing: 10.10.2025
(51) Int. Cl.: C07C 1/24, C07C 11/06

(54) **PRODUCING PROPYLENE FROM ISOPROPANOL**

(30) Priority: 21.10.2024 EP 24306753
(71) Applicant: Technip Energies France, 92741 Nanterre Cedex (FR)
(72) Inventor: Hwang, Shyh-Yuan Henry, 92741 Nanterre Cedex (FR)
(74) Representative: Edson, Russell Gregory

(57) **Abstract**

There is provided a method of producing propylene comprising reacting isopropanol in a feed in a reactor containing an acid catalyst to produce a reactor product comprising propylene, wherein the reactor has a reactor inlet temperature from about 80°C to about 240°C, and separating at least a portion of the propylene from the reactor product.

## Description

### Technical Field

The present disclosure relates to methods for producing propylene. More specifically, but not by way of limitation, this disclosure relates to the production of propylene from a feed comprising isopropanol.

### Background

Olefins, such as ethylene and propylene, have historically been produced by steam or catalytic cracking of hydrocarbons derived from crude oil. Because crude oil is a finite resource, methods for the preparation of olefins by other methods have been proposed. One such method is the catalytic dehydration of alcohols. However, alcohol to olefin reactions can have a high activation energy, which can require high reaction temperatures to achieve reasonable olefin production rates. High reaction temperatures can reduce the lifetime of the catalyst and cause high-carbon number byproducts including aromatics, which reduces the olefin yield and requires additional separation steps. Accordingly, there is a need for an improved and/or alternative process for the production olefins from alcohols.

### Summary

In a first aspect there is provided a method of producing propylene comprising: reacting isopropanol in a feed in a reactor containing an acid catalyst to produce a reactor product comprising propylene, wherein the reactor has a reactor inlet temperature from about 80°C to about 240°C; and separating at least a portion of the propylene from the reactor product.

In an embodiment of the method, the reactor is operated at a reactor inlet temperature at least 10°C above a dew point temperature of a reaction mixture in the reactor and/or at a reactor outlet pressure at least 0.01 MPa below a dew point pressure of the reaction mixture in the reactor.

In an embodiment of the method, the feed further comprises n-propanol at a molar ratio of isopropanol to n-propanol ranging from 10:1 to 1000:1; or
wherein the method comprises introducing the feed into the reactor containing the acid catalyst, the feed comprising isopropanol and optionally n-propanol, wherein, when the n-propanol is included in the feed, a molar ratio of isopropanol to n-propanol ranges from 10:1 to 1000:1, and
reacting the isopropanol and the n-propanol, if present, in the reactor to produce the reactor product comprising propylene.

In an embodiment of the method, the feed comprises the isopropanol and a diluent, optionally wherein a molar ratio of the isopropanol to the diluent ranges from 0.01:1 to 100:1, and/or
wherein the diluent comprises one or more of: hydrogen, nitrogen, steam, methane, ethane, a butane, a pentane, and a hexane.

In an embodiment of the method, the reactor comprises one or more adiabatic reactor units, optionally wherein the reactor comprises multiple adiabatic reactors units in series; or
wherein the reactor comprises one or more isothermal reactor units.

In an embodiment of the method, the acid catalyst comprises an inorganic acid, a silicate, a zeolite, a heteropolyacid, or a mixture thereof, optionally wherein the acid catalyst is a heteropolyacid catalyst.

In an embodiment of the method, the method comprises reacting the isopropanol to produce a reactor product comprising propylene, propane, C4+ hydrocarbons, isopropyl ether, water, and unreacted isopropanol; and
separating the reactor product into a plurality of streams, wherein the plurality of streams comprises a propylene-rich stream, a recycle stream enriched with the isopropyl ether and the unreacted isopropanol, and a byproduct stream comprising at least a portion of the C4+ hydrocarbons and the water.

In an embodiment of the method, the reactor product has a ratio of the amount of propane to propylene of 10,000 (wtppm) or less and a ratio of the amount of ethylene to propylene of 1,000 (wtppm) or less.

In a second aspect, there is provided a method of producing propylene comprising:
introducing a feed into a reactor containing an acid catalyst, the feed comprising isopropanol and optionally n-propanol, wherein, when the n-propanol is included in the feed, a molar ratio of isopropanol to n-propanol ranges from 10:1 to 1000:1, and wherein the reactor has a reactor inlet temperature from 80°C to 240°C;
reacting the isopropanol and the n-propanol, if present, in the reactor to produce a reactor product comprising propylene, propane, C4+ hydrocarbons, isopropyl ether, water, and unreacted isopropanol; and
separating the reactor product into a plurality of streams, wherein the plurality of streams comprises a propylene-rich stream, a recycle stream enriched with the isopropyl ether and the unreacted isopropanol, and a byproduct stream comprising at least a portion of the C4+ hydrocarbons and the water.

In an embodiment of the method of the second aspect, the feed further comprises a diluent, and wherein the reactor product further comprises the diluent, optionally further comprising:
conveying the diluent-rich stream to the reactor, and/or
conveying the recycle stream to the reactor.

In an embodiment of the method of the second aspect, the feed further comprises a diluent and wherein a molar ratio of the isopropanol to the diluent ranges from 0.01:1 to 100:1, optionally wherein the reactor has a reactor inlet temperature from 80°C to 155°C.

In an embodiment of the method, the reactor has a reactor inlet temperature from about 80°C to about 155°C, optionally from about 120°C to about 145°C.

In an embodiment of the method, the reactor inlet temperature is between about 80°C to about 110°C and the method comprises operating the reactor under a reactor outlet gauge pressure of between about 0.05 to about 0.15 MPa; and/or an isopropanol feed weight hour space velocity (WHSV) of between about 0.55 hr⁻¹ to about 0.75 hr⁻¹; and/or have diluent present in the feed at a molar ratio of diluent to isopropanol of between about 7:1 to about 11:1.

In an embodiment of the method, the reactor inlet temperature is between about 170°C to about 210°C and the method comprises operating the reactor under a reactor outlet gauge pressure of between about 0.25 MPa to about 0.5 MPa; and/or an isopropanol feed weight hour space velocity (WHSV) of between about 1.5 hr⁻¹ to about 5 hr⁻¹; and/or have diluent present in the feed at a molar ratio of diluent to isopropanol of between about 5:1 to about 1:1.

### Brief Description of the Drawings

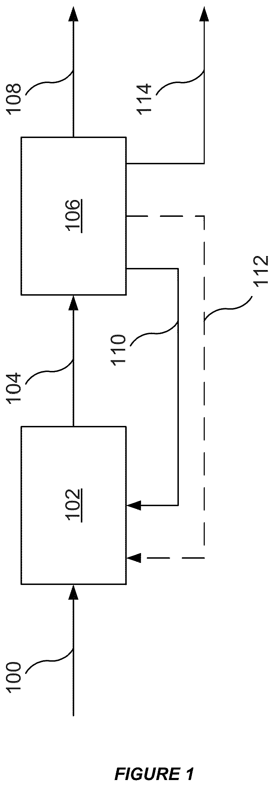

Figure 1 illustrates a process flow diagram for a method of producing propylene according to at least some embodiments of the present disclosure.

### Detailed Description

The present disclosure relates to the production of propylene from a feed comprising isopropanol. Conventionally, n-propanol has been used in alcohol to olefin reactions to produce propylene. The present disclosure uses a feed where the alcohol is primarily isopropanol. Advantageously, it has been identified that isopropanol may be catalytically dehydrated at lower temperatures (e.g., less than 240°C including as low as 80°C) to produce propylene with high yield. The lower temperature may mitigate the production of unwanted byproducts and extend the lifetime of the catalyst. Specific conditions which further optimize the conversion of isopropanol to propylene have also been identified.

Methods of the present disclosure may include reacting isopropanol in the presence of a heteropolyacid catalyst to produce a reactor product comprising propylene; and separating at least a portion of the propylene from the reactor product. Figure 1 illustrates a process flow diagram for a method of producing propylene according to at least some embodiments of the present disclosure. A feed 100 that includes isopropanol and optionally a diluent may be introduced to a reactor 102 containing an acid catalyst. The isopropanol is catalytically dehydrated in the reactor 102 to produce a reactor product 104. The reactor product 104 includes propylene, unreacted isopropanol, diluent, and byproducts (e.g., ethylene, propane, isopropyl ether, C4+ hydrocarbons, and water). The reactor product 104 is then purified in a separator 106 (e.g., one or more distillation columns, one or more membrane separators, and the like) to produce a plurality of streams. In the illustrated example, the plurality of streams includes four streams: a propylene-rich stream 108, a recycle stream 110 enriched with the isopropyl ether and the unreacted isopropanol, a byproduct stream 114 comprising the C4+ hydrocarbons and the water, and a diluent stream 112, which is optional because the use of a diluent is optional. The plurality of streams may include additional streams.

The recycle stream 110 and the diluent stream 112 may be recycled back to the reactor 102. As illustrated, the recycle stream 110 and the diluent stream 112 are recycled separately and directly to the reactor 102. Alternatively, the recycle stream 110 and the diluent stream 112 may be mixed before introduction back into the reactor 102. Alternatively, one or both of the recycle stream 110 and the diluent stream 112 may be mixed with the feed 100 before introduction to the reactor 102.

Accordingly, a method of the present disclosure for producing propylene may include introducing a feed into a reactor containing a heteropolyacid catalyst. The feed may include isopropanol and optionally n-propanol. Then, the method may include reacting the isopropanol and n-propanol, if present, in the reactor to produce a reactor product comprising propylene, propane, ethylene, C4+ hydrocarbons, isopropyl ether, water, and unreacted isopropanol. The method may then include separating the reactor product into a plurality of streams, which may, for example, include a propylene-rich stream, a recycle stream enriched with the isopropyl ether and the unreacted isopropanol, and a byproduct stream comprising the C4+ hydrocarbons and the water. If a diluent is included in the feed, the plurality of streams may further include a diluent-rich stream, depending on the composition of the diluent. Diluents with a lower vaporization temperature (e.g., lower as compared to propylene) may be in a diluent-rich stream, and diluents with a higher vaporization temperature (e.g., higher as compared to propylene) may be in a diluent-rich stream and/or one of the foregoing streams from the plurality of streams.

As used herein, the term "Cn+" refers to compounds composed of hydrogen, n or more number of carbons, and optionally heteroatom(s), excluding isopropyl ether. The compounds may be linear or branched. The compounds may be saturated or unsaturated. For example, C4+ hydrocarbons are compounds with four or more carbons, hydrogen, and optionally, heteroatom(s), excluding isopropyl ether.

The feed may comprise (i) isopropanol, optionally (ii) n-propanol, and optionally (iii) a diluent. The isopropanol and n-propanol may be from any source. For example, isopropanol may be produced by combining water and propene in a hydration reaction; by hydrogenating acetone; by conversion from glucose or glycerol, by microbial fermentation of plants, biomass, algae, waste gas comprising CO and/or CO₂, and/or syngas; and the like.

When n-propanol is present, a molar ratio of isopropanol to n-propanol in the feed may range from 10:1 to 1000:1 (e.g., 10:1 to 100:1, 50:1 to 1000:1, 100:1 to 1000:1, 250:1 to 1000:1, or 500:1 to 1000:1). In some instances, the feed may be at least substantially free of n-propanol where the n-propanol is present at 0.00 mol% to 0.01 mol% relative to the isopropanol.

A diluent may optionally be included in the feed. The diluent should be inert in the reaction of producing propylene from isopropanol. The diluent may serve multiple purposes including to control and/or maintain the dew point of the reaction mixture in the reactor. For example, the reactor may be operated such that the pressure of the reactor is at least 0.01 MPa below the dew point pressure of the reaction mixture and/or the reactor inlet temperature is at least 10°C above the dew point temperature of the reaction mixture. If the conditions in the reactor reach the dew point pressure and dew point temperature of the reaction mixture in the reactor, part of the reaction mixture may condense, causing the reaction to deviate from the desired vapor phase condition.

The inert diluent may allow for higher conversion within the temperature range of the reactor inlet by reducing the endothermic temperature drop caused by the dehydration reaction.

The diluent may be present in the feed at a molar ratio of the isopropanol to the diluent ranging from 0.01:1 to 100:1 (e.g., 0.01:1 to 1:1, or 0.1:1 to 10:1, 0.2:1 to 5:1, 1:1 to 10:1, 1:1 to 100:1, 10:1 to 50:1, or 25:1 to 100:1). In certain embodiments, the diluent to isopropanol ratio is from about 1:1 molar to about 15:1 molar, such as from about 2:1 molar to about 10:1 molar.

Examples of diluents may include, but are not limited to, hydrogen, methane, ethane, a butane (e.g., n-butane and isobutane), a pentane (e.g., n-pentane, isopentane, and neopentane), a hexane (e.g., n-hexane, isohexane, 3-methylpentane, 2,3-dimethylbutane, and 2,2-dimethyl butane), nitrogen, argon, steam, the like, and any combination thereof. In certain embodiments, the diluent is or comprises nitrogen.

The catalyst used in the methods of the present disclosure may include acid catalysts. Examples of acid catalysts may include, but are not limited to, inorganic acids such as tungstic acid, sulphuric acid, phosphoric acid, hydrochloric acid, the like, and any mixture thereof. In another example, the acid catalyst may be, or comprise, a silicate, such as a crystalline silicate or zeolite. In some instances, the acid catalyst may be a crystalline silicate having a Si:Al ratio of at least 100:1 (e.g., at least 150:1, at least 200:1, or at least 250:1), a dealuminated crystalline silicate or a phosphorous modified zeolite.

For example, the acid catalyst may comprise, or may be, a heteropolyacid. Examples of heteropolyacid catalysts may include, but are not limited to, 18-tungstophosphoric acid, 12-tungstophosphoric acid, 12-molybdophosphoric acid, 12-tungstosilicic acid, 12-molybdosilicic acid, cesium hydrogen tungstosilicate, the like, and any combination thereof. Examples of heteropolyacid catalyst may include the free acid or partial salts of heteropolyacids including, but not limited to, monopotassium tungstophosphate, monosodium 12-tungstosilicic acid, potassium tungstophosphate, sodium molybdophosphate, ammonium molybdodiphosphate, potassium molybdodivanado phosphate, the like, and any combination thereof. In some instances, the heteropolyacid catalyst (e.g., silicotungstic acid) may be supported on a suitable inert support, such as silica or alumina.

The reactor may be a single reactor or a plurality of reactor units in series and/or parallel. The total number of reactor units may range from 1 to 10 or more (e.g., 1 to 3, 2 to 5, or 4 to 10). Each reactor unit may be an isothermal reactor or an adiabatic reactor. In one example, the reactor may include one or more adiabatic reactor units. In another example, the reactor include multiple adiabatic reactor units in series. In yet another example, the reactor may include one or more isothermal reactor units. In another example, the reactor may include multiple isothermal reactors in series. In yet another example, the reactor may include multiple isothermal reactors in parallel.

The reactor may be operated at a reactor inlet temperature ranging from 80°C to 240°C (e.g., 80°C to 200°C, 80°C to 180°C, 80°C to 155°C, or 80°C to 150°C). In certain embodiments, the reactor may be operated at a reactor inlet temperature ranging from 80°C to 155°C, optionally from 100°C to 155°C, optionally from 110°C to 155°C, optionally from 120°C to 155°C, optionally from 130°C to 155°C, or from 80°C to 145°C, optionally from 90°C to 145°C, optionally from 100°C to 145°C, optionally from 110°C to 145°C, optionally from 120°C to 145°C, optionally from 130°C to 145°C, or from 80°C to 140°C, optionally from 90°C to 140°C, optionally from 100°C to 140°C, optionally from 110°C to 140°C, optionally from 120°C to 140°C, optionally from 130°C to 140°C, or from 100°C to 240°C, optionally from 120°C to 240°C, optionally from 120°C to 200°C, optionally from 120°C to 180°C, optionally from 120°C to 160°C. The reactor may be operated at a reactor outlet pressure ranging from 0.01 MPa absolute (MPa-a) to 30 MPa-a (e.g., 0.03 MPa-a to 20 MPa-a, 0.1 MPa-a to 15 MPa-a, or 0.2 MPa-a to 10 MPa-a). The reactor may be operated at an isopropanol feed weight hour space velocity (WHSV) of 0.01 hr⁻¹ to 100 hr⁻¹ (e.g., 0.01 hr⁻¹ to 10 hr⁻¹, 0.01 hr⁻¹ to 50 hr⁻¹, 0.1 hr⁻¹ to 100 hr⁻¹, 0.1 hr⁻¹ to 5 hr⁻¹, 0.1 hr⁻¹ to 20 hr⁻¹, 0.1 hr⁻¹ to 50 hr⁻¹, or 1 hr⁻¹ to 100 hr⁻¹). The isopropanol feed weight hour space velocity (WHSV) is calculated by dividing the isopropanol feed rate in Kg/hr, by the weight of the catalyst in Kg. In certain embodiments, the reactor outlet pressure is from about 0 MPa gauge to about 1.0 MPa gauge, and the isopropanol WHSV is from about 0.1 hr⁻¹ to about 10 hr⁻¹. In certain embodiments, the reactor outlet pressure is from about 0.05 MPa gauge to about 0.4 MPa gauge, such as from about 0.1 MPa gauge to about 0.2 MPa gauge. In certain embodiments, the isopropanol WHSV is from about 0.1 hr⁻¹ to about 2 hr⁻¹, such as from about 0.5 hr⁻¹ to about 1 hr⁻¹.

As discussed above, the reactor may be operated such that the reactor pressure is at least 0.01 MPa below (e.g., at least 0.02 MPa below, 0.01 MPa to 0.5 MPa below, or 0.02 MPa to 1 MPa below) the dew point pressure of the reaction mixture and/or the reactor inlet temperature is at least 10°C above (e.g., at least 20°C above, 10°C to 30°C above, 10°C to 50°C above, or 10°C to 100°C above) the dew point temperature of the reaction mixture. Dew point pressure is a thermodynamic property of the process stream that depends on the temperature and composition of said process stream. Dew point temperature is a thermodynamic property of the process stream that depends on the pressure and composition of said process stream. Both the dew point pressure and dew point temperature are preferably calculated by chemical process simulators such as Aspen Plus, PD-Plus, and CHEMCAD.

The product from the reactor (also referred to herein as the reactor product) may include propylene, unreacted isopropanol, diluent, and byproducts (e.g., propane, ethylene, isopropyl ether, C4+ hydrocarbons, and water). Advantageously, the lower temperatures in the reactor for the methods of the present disclosure (as compared to temperatures for converting n-propanol to propylene) may reduce the amount of byproducts produced.

The ratio of the amount of two components may be presented herein as wtppm. A ratio of Component A to Component B reported as wtppm is (the weight of Component A in the reactor product) / (the weight of Component B in the reactor product) * 1,000,000.

A ratio of the amount of propane to propylene in the reactor product may be 10,000 (wtppm) or less (e.g., less than 5,000 (wtppm), less than 2,000 (wtppm), less than 1,000 (wtppm), less than 500 (wtppm), 0 (wtppm) to 10,000 (wtppm), 0 (wtppm) to 5,000 (wtppm), 0 (wtppm) to 2,000 (wtppm), 0 (wtppm) to 1,000 (wtppm), or 0 (wtppm) to 500 (wtppm)). Conventional processes of converting isopropanol to propylene often have over 10,000 (wtppm) of propane to propylene in the reactor product.

A ratio of the amount of ethylene to propylene in the reactor product may be 1,000 (wtppm) or less (e.g., less than 500 (wtppm), less than 250 (wtppm), less than 100 (wtppm), less than 50 (wtppm), 0 (wtppm) to 1,000 (wtppm), 0 (wtppm) to 500 (wtppm), 0 (wtppm) to 250 (wtppm), 0 (wtppm) to 100 (wtppm), or 0 (wtppm) to 50 (wtppm)).

A ratio of the amount of C4 hydrocarbons to propylene in the reactor product may range from 10 (wtppm) to 950 (wtppm) (e.g., 50 (wtppm) to 500 (wtppm), 75 (wtppm) to 750 (wtppm), or 50 (wtppm) to 950 (wtppm)).

A ratio of the amount of C4+ hydrocarbons to propylene in the reactor product may be 2 (wt%) or less (e.g., less than 1 (wt%), less than 0.5 (wt%), less than 0.3 (wt%), 0 (wt%) to 1 (wt%), 0 (wt%) to 0.5 (wt%), or 0 (wt%) to 0.3 (wt%)).

The reactor product may be separated into a plurality of streams. Separation may be via distillation and/or membrane separation.

Separation may result in a plurality of streams including a propylene-rich stream, which may contain propylene at a concentration of at least 95 wt% (e.g., at least 98 wt%, at least 99 wt%, at least 99.5 wt%, or at least 99.7 wt%), based on a total weight of the propylene-rich stream.

Other streams may include a recycle stream enriched with the isopropyl ether and the unreacted isopropanol, which may be conveyed back to the reactor to produce propylene. Another stream may be a byproduct stream comprising the C4+ hydrocarbons and the water. Yet another stream may be a diluent stream, which is especially applicable if the diluent has a lower vaporization temperature than propylene, such as hydrogen, methane, ethane, and nitrogen. Heavier diluents may also be in a separate diluent stream or may be in the recycle stream.

While it has been found that the conversion can take place at lower temperatures, it has also been surprisingly found that the output can be further optimized at particular temperatures by adjusting certain reaction conditions. The general methods described herein are not intended to be limited to requiring these adjusted certain reaction conditions but merely that the reaction output can optionally be further optimized within particular temperature ranges by the adjustment of these certain reaction conditions.

In this respect, it has surprisingly been found that at lower reactor inlet temperatures within the 80-240°C range, the conversion to propylene can be further optimized by adjusting the (1) reactor outlet pressure and/or (2) diluent to isopropanol feed molar ratio and/or (3) isopropanol feed WHSV to within certain ranges.

In this respect, at lower reactor inlet temperature ranges such as from 80°C to 145°C, optionally from 80°C to 135°C, optionally from 80°C to 125°C, optionally from 80°C to 115°C, optionally from 80°C to 110°C, optionally from 80°C to 105°C, optionally from 80°C to 100°C, it has been found advantageous to operate the reactor under one or more (or all) of the following conditions: a reactor outlet gauge pressure lower than 0.2 MPa, optionally lower than 0.19 MPa, optionally lower than 0.15 MPa, optionally lower than 0.11 MPa, such as between about 0.05 MPa to about 0.15 MPa, or between about 0.08 MPa to about 0.12 MPa; and/or an isopropanol feed weight hour space velocity (WHSV) of less than 0.8 hr⁻¹, optionally less than about 0.7 hr⁻¹, such as between about 0.55 hr⁻¹ to about 0.75 hr⁻¹, or between about 0.6 hr⁻¹ to about 0.7 hr⁻¹; and/or diluent may be present in the feed at a molar ratio of diluent to isopropanol of higher than 3:1, optionally higher than 4:1, optionally higher than 5:1, optionally higher than 6:1, optionally higher than 7:1, optionally higher than 8:1, such as between about 6:1 to about 12:1, or between about 7:1 to about 11:1, or between about 8:1 to about 10:1. Most preferred at a temperature range of between about 80°C to about 110°C is to operate the reactor under a reactor outlet gauge pressure of between about 0.05 to about 0.15 MPa, such as between about 0.08 to about 0.12 MPa; an isopropanol feed weight hour space velocity (WHSV) of between about 0.55 hr⁻¹ to about 0.75 hr⁻¹, such as between about 0.6 hr⁻¹ to about 0.7 hr⁻¹; and have diluent present in the feed at a molar ratio of diluent to isopropanol of between about 7:1 to about 11:1, such as between about 8:1 to about 10:1. These conditions were found to increase the conversion to propylene, while maintaining low amounts of propane and heavies.

It has also surprisingly been found that at higher reactor inlet temperatures within the 80-240°C range, the conversion to propylene can be further enhanced by adjusting the reactor outlet pressure and/or isopropanol feed WHSV to within certain ranges.

At higher reactor inlet temperature ranges such as from 170°C to 240°C, optionally from 180°C to 220°C, optionally from 180°C to 200°C, it has been found advantageous to operate the reactor under one or more (or all) of the following conditions: a reactor outlet gauge pressure higher than 0.2 MPa, optionally higher than 0.25 MPa, optionally higher than 0.3 MPa, optionally higher than 0.4 MPa, such as between about 0.25 MPa to about 0.5 MPa, or between about 0.3 MPa to about 0.4 MPa; and/or an isopropanol feed weight hour space velocity (WHSV) of higher than 0.9 hr⁻¹, optionally higher than about 1.5 hr⁻¹, or higher than about 3 hr⁻¹, such as between about 1.5 hr⁻¹ to about 5 hr⁻¹, or between about 1.8 hr⁻¹ to about 4 hr⁻¹, or between about 1.5 hr⁻¹ to about 2 hr⁻¹ or between about 3 hr⁻¹ to about 5 hr⁻¹. Diluent may be present in the feed at a molar ratio of diluent to isopropanol of between about 5:1 to about 1:1. Most preferred at a temperature range of between about 170°C to about 210°C, such as between about 180°C to about 200°C is to operate the reactor under a reactor outlet gauge pressure of between about 0.25 MPa to about 0.5 MPa, such as between about 0.3 MPa to about 0.4 MPa; and an isopropanol feed weight hour space velocity (WHSV) of between about 1.5 hr⁻¹ to about 5 hr⁻¹, such as between about 1.8 hr⁻¹ to about 4 hr⁻¹. These conditions were found to reduce the amounts of propane and heavies formed, while maintaining high conversion to propylene.

### Examples 1 and 2

A reactor containing a heteropolyacid catalyst was used for converting isopropanol to propylene. The feed was isopropanol diluted with nitrogen with a 3:1 molar ratio of nitrogen to isopropanol. The reactor outlet pressure was 0.2 MPa gauge, and the isopropanol feed WHSV was 0.86 hr⁻¹. Table 1 provides the reactor temperature and the composition of the reactor product before purification.

**Table 1**

| Example | Reactor Inlet Temp. (°C) | Conversion to Propylene | Ethylene / Propylene (wtppm) | Propane / Propylene (wtppm) | C4 / Propylene (wtppm) | Heavies* / Propylene (wt%) |
|---|---|---|---|---|---|---|
| 1 | 130 | 48% | < 10 | 150 | 170 | 0.2 |
| 2 | 140 | 68% | < 10 | 160 | 110 | 0.2 |

| | | | | | | |
|---|---|---|---|---|---|---|
| * Heavies are C4+ compounds excluding isopropyl ether. | | | | | | |

The expected carbon efficiency for each of the foregoing examples was 99.5% to 99.8%.

### Examples 3 to 12

A computational model was developed and used to project the reactor performance at different inlet temperatures. Table 2 provides the composition of the reactor product before purification at selected reactor inlet temperatures. The projected composition at 130 and 140°C reactor inlet temperature in Table 2 matches with the experimental results in Table 1, confirming accuracy of the computational model.

**Table 2**

| Example | Reactor Inlet Temp. (°C) | Conversion to Propylene | Ethylene / Propylene (wtppm) | Propane / Propylene (wtppm) | C4 / Propylene (wtppm) | Heavies* / Propylene (wt%) |
|---|---|---|---|---|---|---|
| 3 | 80 | 9% | < 10 | 80 | 1050 | <0.1 |
| 4 | 100 | 18% | < 10 | 100 | 500 | <0.1 |
| 5 | 120 | 35% | < 10 | 130 | 240 | 0.1 |
| 6 | 130 | 48% | < 10 | 150 | 170 | 0.2 |
| 7 | 140 | 68% | < 10 | 160 | 110 | 0.2 |
| 8 | 150 | 94% | < 10 | 190 | 80 | 0.4 |
| 9 | 155 | >99% | < 10 | 200 | 70 | 0.5 |
| 10 | 180 | >99% | < 10 | 270 | 30 | 1.4 |
| 11 | 200 | >99% | < 10 | 340 | 10 | 3.5 |
| 12 | 240 | >99% | < 10 | 560 | <10 | 20.8 |

| | | | | | | |
|---|---|---|---|---|---|---|
| * Heavies are C4+ compounds excluding isopropyl ether. | | | | | | |

Table 2 shows that the reaction can be carried out at temperatures between 80 and 240°C with varying performance. When the reaction is carried out at lower temperatures, lower amounts of propane and heavies are produced, but the process faces a reduced conversion rate and higher amounts of C4 are generated. When the reaction is carried out at higher temperatures, there is higher conversion and less C4 made but higher amounts of propane and heavies are generated. Therefore, the reactor inlet temperature can be chosen based on the desired reaction performance. However, surprisingly, it was found that within the range from about 120 to about 145°C, a reactor inlet temperature was found which excellently balances propane and heavies production, with conversion rate and C4 production, for the isopropanol dehydration reaction to propylene.

It has also surprisingly been found possible to further optimize the reaction performance by adjusting other reaction conditions such as reactor outlet pressure, isopropanol feed WHSV, and/or feed diluent to isopropanol ratio. For example, the conversion to propylene at lower reactor inlet temperatures (such as from about 80°C to about 145°C, particularly from about 80°C to about 110°C)can be increased by increasing diluent to isopropanol feed ratio (i.e., nitrogen to isopropanol ratio) and/or reducing the reactor outlet pressure and/or reducing the isopropanol feed WHSV. For example, it was found that the 9% conversion in Example 3 can be raised to above 20% and the 18% conversion in Example 4 can be raised to above 40%, while maintaining low amounts of propane and heavies, by increasing the nitrogen to isopropanol ratio from 3:1 molar to 9:1 molar, reducing reactor outlet pressure from 0.2 MPa gauge to 0.1 MPa gauge and reducing feed WHSV from 0.86 hr⁻¹ to 0.65 hr⁻¹. It was also surprisingly found that the high propane and heavies produced at higher reactor inlet temperatures (such as from about 150°C to about 240°C, particularly from about 180°C to about 210°C) could be reduced, while maintaining high conversion levels, by increasing the reactor outlet pressure and/or the feed WHSV. For example, the 1.4 wt% of heavies produced at 180°C in Example 10 could be reduced to below 0.7 wt%, while maintaining 100% conversion, by increasing the feed WHSV from 0.86 hr⁻¹ to 1.8 hr⁻¹. Similarly, the 3.5 wt% of heavies produced at 200°C in Example 11 could be reduced to below 0.9 wt%, while maintaining 100% conversion, by increasing the feed WHSV from 0.86 hr⁻¹ to 4 hr⁻¹.

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure. For instance, any examples described herein can be combined with any other examples to yield further examples.

The present method will also be described by the following numbered embodiments:
1. A method of producing propylene comprising:
   reacting isopropanol in a feed in a reactor containing an acid catalyst to produce a reactor product comprising propylene; and
   separating at least a portion of the propylene from the reactor product.
2. The method of embodiment 1, wherein the reactor has a reactor inlet temperature from 80°C to 240°C.
3. The method of embodiment 1, wherein the reactor has a reactor inlet temperature from 80°C to 200°C.
4. The method of embodiment 1, wherein the reactor has a reactor inlet temperature from 80°C to 155°C.
5. The method of embodiment 1, wherein the reactor has a reactor outlet pressure from 0.01 MPa absolute to 30 MPa absolute.
6. The method of embodiment 1, wherein the reactor is operated at a reactor inlet temperature at least 10°C above a dew point temperature of a reaction mixture in the reactor and/or at a reactor outlet pressure at least 0.01 MPa above a dew point pressure of the reaction mixture in the reactor.
7. The method of embodiment 1, wherein the feed further comprises n-propanol at a molar ratio of isopropanol to n-propanol ranging from 10:1 to 1000:1.
8. The method of embodiment 1, wherein the feed comprises the isopropanol and a diluent.
9. The method of embodiment 8, wherein a molar ratio of the isopropanol to the diluent ranges from 0.01:1 to 100:1.
10. The method of embodiment 8, wherein the diluent comprises one or more of: hydrogen, nitrogen, steam, methane, ethane, a butane, a pentane, and a hexane.
11. The method of embodiment 1, wherein the acid catalyst comprises an inorganic acid, a silicate, a zeolite, a heteropolyacid, or a mixture thereof.
12. The method of embodiment 1, wherein the reactor comprises one or more adiabatic reactor units.
13. The method of embodiment 1, wherein the reactor comprises multiple adiabatic reactors units in series.
14. The method of embodiment 1, wherein the reactor comprises one or more isothermal reactor units.
15. A method of producing propylene comprising:
   introducing a feed into a reactor containing an acid catalyst, the feed comprising isopropanol and optionally n-propanol, wherein, when the n-propanol is included in the feed, a molar ratio of isopropanol to n-propanol ranges from 10:1 to 1000:1, and wherein the reactor has a reactor inlet temperature from 80°C to 240°C;
   reacting the isopropanol and the n-propanol, if present, in the reactor to produce a reactor product comprising propylene, propane, C4+ hydrocarbons, propyl ether (isopropyl ether), water, and unreacted isopropanol; and
   separating the reactor product into a plurality of streams, wherein the plurality of streams comprises a propylene-rich stream, a recycle stream enriched with the propyl ether (isopropyl ether) and the unreacted isopropanol, and a byproduct stream comprising at least a portion of the C4+ hydrocarbons and the water.
16. The method of embodiment 15, wherein the feed further comprises a diluent, and wherein the reactor product further comprises the diluent.
17. The method of embodiment 16 further comprising:
   conveying the diluent-rich stream to the reactor.
18. The method of embodiment 15 further comprising:
   conveying the recycle stream to the reactor.
19. The method of embodiment 15, wherein the reactor has a reactor inlet temperature from 80°C to 200°C.
20. A method of producing propylene comprising:
   introducing a feed into a reactor containing an acid catalyst, the feed comprising isopropanol, a diluent, and optionally n-propanol, wherein, when the n-propanol is included in the feed, a molar ratio of isopropanol to n-propanol ranges from 10:1 to 1000:1, wherein a molar ratio of the isopropanol to the diluent ranges from 0.01:1 to 100:1, and wherein the reactor has a reactor inlet temperature from 80°C to 155°C;
   reacting the isopropanol and n-propanol, if present, in the reactor to produce a reactor product comprising propylene, propane, C4+ hydrocarbons, propyl ether (isopropyl ether), water, and unreacted isopropanol; and
   separating the reactor product into a plurality of streams, wherein the plurality of streams comprises a propylene-rich stream, a recycle stream enriched with the propyl ether (isopropyl ether) and the unreacted isopropanol, and a byproduct stream comprising at least a portion of the C4+ hydrocarbons and the water.

## Claims

1. A method of producing propylene comprising:
reacting isopropanol in a feed in a reactor containing an acid catalyst to produce a reactor product comprising propylene, wherein the reactor has a reactor inlet temperature from about 80°C to about 240°C; and
separating at least a portion of the propylene from the reactor product.

2. The method of claim 1, wherein the reactor is operated at a reactor inlet temperature at least 10°C above a dew point temperature of a reaction mixture in the reactor and/or at a reactor outlet pressure at least 0.01 MPa below a dew point pressure of the reaction mixture in the reactor.

3. The method of claims 1 or 2, wherein the feed further comprises n-propanol at a molar ratio of isopropanol to n-propanol ranging from 10:1 to 1000:1; or
wherein the method comprises introducing the feed into the reactor containing the acid catalyst, the feed comprising isopropanol and optionally n-propanol, wherein, when the n-propanol is included in the feed, a molar ratio of isopropanol to n-propanol ranges from 10:1 to 1000:1, and
reacting the isopropanol and the n-propanol, if present, in the reactor to produce the reactor product comprising propylene.

4. The method of any preceding claim, wherein the feed comprises the isopropanol and a diluent, optionally wherein a molar ratio of the isopropanol to the diluent ranges from 0.01:1 to 100:1, and/or
wherein the diluent comprises one or more of: hydrogen, nitrogen, steam, methane, ethane, a butane, a pentane, and a hexane.

5. The method of any preceding claim, wherein the reactor comprises one or more adiabatic reactor units, optionally wherein the reactor comprises multiple adiabatic reactors units in series.

6. The method of any of claims 1 to 4, wherein the reactor comprises one or more isothermal reactor units.

7. The method of any preceding claim, wherein the acid catalyst comprises an inorganic acid, a silicate, a zeolite, a heteropolyacid, or a mixture thereof, optionally wherein the acid catalyst is a heteropolyacid catalyst.

8. The method of any preceding claim, wherein the method comprises reacting the isopropanol to produce a reactor product comprising propylene, propane, C4+ hydrocarbons, isopropyl ether, water, and unreacted isopropanol; and
separating the reactor product into a plurality of streams, wherein the plurality of streams comprises a propylene-rich stream, a recycle stream enriched with the isopropyl ether and the unreacted isopropanol, and a byproduct stream comprising at least a portion of the C4+ hydrocarbons and the water.

9. The method of any preceding claim, wherein the reactor product has a ratio of the amount of propane to propylene of 10,000 (wtppm) or less and a ratio of the amount of ethylene to propylene of 1,000 (wtppm) or less.

10. A method of producing propylene comprising:
introducing a feed into a reactor containing an acid catalyst, the feed comprising isopropanol and optionally n-propanol, wherein, when the n-propanol is included in the feed, a molar ratio of isopropanol to n-propanol ranges from 10:1 to 1000:1, and wherein the reactor has a reactor inlet temperature from 80°C to 240°C;
reacting the isopropanol and the n-propanol, if present, in the reactor to produce a reactor product comprising propylene, propane, C4+ hydrocarbons, isopropyl ether, water, and unreacted isopropanol; and
separating the reactor product into a plurality of streams, wherein the plurality of streams comprises a propylene-rich stream, a recycle stream enriched with the isopropyl ether and the unreacted isopropanol, and a byproduct stream comprising at least a portion of the C4+ hydrocarbons and the water.

11. The method of claim 10, wherein the feed further comprises a diluent, and wherein the reactor product further comprises the diluent, optionally further comprising:
conveying the diluent-rich stream to the reactor, and/or
conveying the recycle stream to the reactor.

12. The method of claims 10 or 11, wherein the feed further comprises a diluent and wherein a molar ratio of the isopropanol to the diluent ranges from 0.01:1 to 100:1, optionally wherein the reactor has a reactor inlet temperature from 80°C to 155°C.

13. The method of any preceding claim, wherein the reactor has a reactor inlet temperature from about 80°C to about 155°C, optionally from about 120°C to about 145°C.

14. The method of any preceding claim, wherein the reactor inlet temperature is between about 80°C to about 110°C and the method comprises operating the reactor under a reactor outlet gauge pressure of between about 0.05 to about 0.15 MPa; and/or an isopropanol feed weight hour space velocity (WHSV) of between about 0.55 hr⁻¹ to about 0.75 hr⁻¹; and/or have diluent present in the feed at a molar ratio of diluent to isopropanol of between about 7:1 to about 11:1.

15. The method of any of claims 1 to 12, wherein the reactor inlet temperature is between about 170°C to about 210°C and the method comprises operating the reactor under a reactor outlet gauge pressure of between about 0.25 MPa to about 0.5 MPa; and/or an isopropanol feed weight hour space velocity (WHSV) of between about 1.5 hr⁻¹ to about 5 hr⁻¹; and/or have diluent present in the feed at a molar ratio of diluent to isopropanol of between about 5:1 to about 1:1.
